# EUROPÄISCHE PATENTSCHRIFT

(11) **EP 1 920 105 B1**
(45) Veröffentlichungstag und Bekanntmachung des Hinweises auf die Patenterteilung: **25.03.2015**
(21) Anmeldenummer: 06792747.5
(22) Anmeldetag: 09.08.2006
(51) Int. Cl.: D06M 15/263, D06M 13/148, D06M 13/17, D06M 11/13, D06M 11/48, D06M 11/56, D06M 11/64, D06M 15/21, A61F 13/00, D06N 3/04, C08J 7/04, A61F 13/53, C08J 5/04, D06N 3/00

(54) **VERBESSERTE FEUCHTIGKEITSREGULIERENDE VERBUNDSTOFFE**
IMPROVED HUMIDITY-REGULATING COMPOSITE MATERIALS
MATERIAUX COMPOSITES REGULATEURS D'HUMIDITE AMELIORES

(30) Priorität: 23.08.2005 DE 102005039968
(43) Veröffentlichungstag der Anmeldung: 14.05.2008
(73) Patentinhaber: BASF SE, 67056 Ludwigshafen (DE)
(72) Erfinder: HERRLICH-LOOS, Mirjam, 68165 Mannheim (DE); HAINDL, Corinna, 68163 Mannheim (DE); CHAMP, Samantha, 67063 Ludwigshafen (DE); BECK, Martin, 67133 Maxdorf (DE); TÖNNESSEN, Markus, 67069 Ludwigshafen (DE); FASTNER, Michael, 68239 Mannheim (DE)
(86) Internationale Anmeldenummer: PCT/EP2006/065170
(87) Internationale Veröffentlichungsnummer: WO 2007/023086

(56) Entgegenhaltungen:
- DE-A1- 4 127 337
- GB-A- 2 376 695
- DATABASE WPI Week 199321 Derwent Publications Ltd., London, GB; AN 1993-172678 XP002387973 & JP 05 105705 A (NIPPON SHOKUBAI CO LTD) 27. April 1993 (1993-04-27) in der Anmeldung erwähnt

## Beschreibung

Die vorliegende Erfindung betrifft verbesserte feuchtigkeitsregulierende Verbundstoffe, Verfahren zu ihrer Herstellung sowie deren Verwendung zur Feuchtigkeitsregulierung.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Der Stand der Technik zur Herstellung wasserabsorbierender Polymere wird beispielsweise in der Monographie "Modern Superabsorbent Polymer Technology", F.L. Buchholz und A.T. Graham, Wiley-VCH, 1998, Seiten 69 bis 117, zusammenfassend beschrieben.

WO 01/56625, EP-A 1 178 149 und US 5,962,068 beschreiben Verfahren zur Herstellung wasserabsorbierender Verbundstoffe, bei denen wasserabsorbierende Polymere auf ein Trägermaterial aufpolymerisiert werden.

Die WO 00/64311 offenbart Verbundstoffe bei denen wasserabsorbierende Polymere auf ein Trägermaterial polymerisiert wurden. Die Verbundstoffe werden zur Feuchtigkeitsregulierung in Sitzpolstern verwendet. WO 2004/067826 A1 lehrt mehrschichtige textile Flächengebilde, insbesondere solche aus einseitig maschenbeschichteten Vliesstoffen, die Funktionsmittel wie zum Beispiel wasserabsorbierende Polymere enthaften können.

JP-A 05-105705 betrifft nichtzerfließende Trockenmittel, bestehend aus einem Trägermaterial und hygroskopischen Salzen, wobei die hygroskopischen Salze mittels wasserabsorbierender Polymere auf dem Trägermaterial fixiert werden.

Die ältere deutsche Patentanmeldung mit dem Aktenzeichen DE 10 2005 015 536.7 beschreibt feuchtigkeitsregulierende Verbundstoffe, die mindestens ein flächiges Trägermaterial, mindestens eine wasserlösliche hygroskopische Substanz und mindestens ein in Gegenwart der wasserlöslichen hygroskopischen Substanz auf das Trägermaterial aufpolymerisiertes wasserabsorbierendes Polymer enthalten.

Die bekannten feuchtigkeitsregulierenden Verbundstoffe sind häufig relativ steif und die enthaltenen Partikel wasserabsorbierender Polymere sind relativ hart. Bei der Weiterverarbeitung und Verwendung der Verbundstoffe können durch diese Steifheit Schwierigkeiten auftreten, die Drapierfähigkeit der Verbundstoffe leidet. Zudem können sich bei lokalem Kontakt derartiger feuchtigkeitsregulierender Verbundstoffe - die meist zur Vermeidung mit Transpiration verbundener unangenehmer Effekte verwendet werden - mit größeren Mengen Flüssigkeit als gängigerweise durch Transpiration erzeugt wird (beispielsweise beim Verschütten von Flüssigkeit auf derart ausgestatte Sitzmöbel oder Autositze) unerwünschte Unebenheiten bilden, deren Rückbildung durch Trocknung länger dauert als das übliche Trocknen der feuchtigkeitsregulierenden Verbundstoffe.

Aufgabe der vorliegenden Erfindung war daher die Bereitstellung feuchtigkeitsregulierender Verbundstoffe mit guter Drapierfähigkeit.

Gelöst wurde die Aufgabe der vorliegenden Erfindung durch feuchtigkeitsregulierende Verbundstoffe, umfassend
a) mindestens ein flächiges Trägermaterial,
b) mindestens eine wasserlösliche hygroskopische Substanz und
c) mindestens ein in Gegenwart der Substanz b) auf das Trägermaterial a) aufpolymerisiertes wasserabsorbierendes Polymer, wobei das Polymer als Vernetzer Verbindungen mit mindestens zwei polymerisierbaren Gruppen aufweist, die radikalisch einpolymerisiert werden können, und
wobei das Gewichtsverhältnis von hygroskopischer Substanz b) zu Polymer c) im Bereich von 0,01 und 1 liegt, die dadurch gekennzeichnet sind, dass das Polymer c) einen Weichmacher enthält.

Das Verhältnis von hygroskopischer Substanz b) zu Polymer c) beträgt vorzugsweise weniger als 0,8, bevorzugt weniger als 0,6, besonders bevorzugt weniger als 0,5, ganz besonders bevorzugt weniger als 0,4, und mindestens 0,05, bevorzugt mindestens 0,1, besonders bevorzugt mindestens 0,15.

Die Trägermaterialien a) unterliegen keiner Beschränkung. Bevorzugte Trägermaterialien sind Gewebe und/oder Vliese, wie in der WO 01/56625 auf Seite 16, Zeile 40, bis Seite 20, Zeile 27, beschrieben werden, oder Mischformen aus Geweben und Vliesen wie beispielsweise in WO 2004/067826 offenbart.

Geeignete Trägermaterialien a) sind beispielsweise Gewebe oder Vliese aus synthetischen polymeren Fasern. Die Fasern können aus jedem spinnbaren polymeren Material sein, beispielsweise Polyolefine, wie Polyethylen oder Polypropylen, Polyester, wie Polyethylenterephthalat, Polyamide, wie Nylon® 6 oder Nylon® 6,6, Polyacrylate, modifizierte Zellulosen, wie Zelluloseacetat. Weiterhin können auch Gemische obengenannter polymeren Materialien eingesetzt werden.

Gewebe sind Erzeugnisse aus gekreuzten Fasern, vorzugsweise rechtwinklig gekreuzten Fasern.

Vliese sind nicht gewebte Erzeugnisse aus Fasern, bei denen der Zusammenhalt im allgemeinen durch die den Fasern eigene Haftung gegeben ist. Vorzugsweise werden Vliese mechanisch verfestigt, beispielsweise durch Vernadeln, Vermaschen oder Verwirbeln mittels scharfer Wasser- oder Luftstrahlen. Vliese können auch adhäsiv oder kohäsiv verfestigt werden. Adhäsiv verfestigte Vliese sind beispielsweise erhältlich durch Verkleben der Fasern mit flüssigen Bindemitteln oder durch Schmelzen von Bindefasern, die dem Vlies bei der Herstellung zugesetzt wurden. Kohäsiv verfestigte Vliese sind beispielsweise durch Anlösen der Fasern mit geeigneten Chemikalien und Anwendung von Druck.

Die Trägermaterialien weisen zweckmäßigerweise ein Flächengewicht von 20 bis 800 g/m², vorzugsweise von 50 bis 600 g/m², besonders bevorzugt von 100 bis 500 g/m², auf.

Hygroskopische Substanzen b) sind Stoffe, die in der Lage sind Wasserdampf zu absorbieren, d.h., Wasserdampf aus der Luft kondensiert auf der hygroskopischen Substanz, wobei der Wassergehalt der hygroskopischen Substanz b) steigt. Hygroskopische Substanzen b) sind beispielsweise anorganische Salze, wie Natriumchlorid, Bleinitrat, Zinksulfat, Natriumperchlorat, Chromoxid oder Lithiumchlorid, oder zumindest teilkristalline organische Verbindungen, wie wasserlösliche Polyacrylsäuren. Hygroskopische anorganische Salze sind bevorzugte hygroskopische Substanzen b). Ganz besonders bevorzugt ist Natriumchlorid.

Besonders vorteilhafte hygroskopische Substanzen b) sind Verbindungen, bei denen sich über einer gesättigten wässrigen Lösung bei 20°C im Gleichgewicht eine relative Feuchte von weniger als 95%, vorzugsweise weniger als 90%, bevorzugt weniger als 85%, besonders bevorzugt weniger als 80%, und von mindestens 40%, vorzugsweise mindesten 45%, bevorzugt mindestens 50%, besonders bevorzugt mindestens 55%, ganz besonders bevorzugt mindesten 60%, einstellt.
Die relative Feuchte ist der Quotient aus Wasserdampfpartialdruck und Wasserdampfdruck multipliziert mit 100%.

Vorzugsweise liegt die hygroskopische Substanz b) im aufpolymerisierten wasserabsorbierenden Polymer verteilt vor.

Bei der Verwendung der erfindungsgemäßen feuchtigkeitsregulierenden Verbundstoffe, beispielsweise in Sitzpolstern, werden relative Feuchten von über 90% als unangenehm empfunden, da bei hoher Luftfeuchtigkeit die Schweißbildung begünstigt wird. Relative Feuchten unter 40% sind aber auch nicht vorteilhaft, da derart niedrige relative Feuchten den Sitzkomfort nicht weiter erhöhen.

Die erfindungsgemäßen feuchtigkeitsregulierenden Verbundstoffe können durch Polymerisation einer Monomerlösung, enthaltend
i) mindestens ein ethylenisch ungesättigtes Monomer,
ii) mindestens eine wasserlösliche hygroskopische Substanz,
iii) einen Vernetzer, der mindestens zwei polymerisierbare Gruppen aufweist, die radikalisch einpolymerisiert werden können, und
iv) gegebenenfalls ein oder mehrere mit den unter i) genannten Monomeren copolymerisierbare ethylenisch und/oder allylisch ungesättigte Monomere
welche auf ein Trägermaterial aufgebracht und polymerisiert wird, erhalten werden, wobei das Gewichtsverhältnis von hygroskopischer Substanz ii) zu Monomer i) im Bereich von 0,01 bis 1 liegt, das dadurch gekennzeichnet ist, dass der Monomerlösung zusätzlich ein Weichmacher zugesetzt wird.

Das Gewichtsverhältnis von hygroskopischer Substanz ii) zu Monomer i) beträgt vorzugsweise höchstens, insbesondere weniger als 0,8, bevorzugt höchstens, insbesondere weniger als 0,6, besonders bevorzugt höchstens, insbesondere weniger als 0,5, ganz besonders bevorzugt höchstens, insbesondere weniger als 0,4, und mindestens 0,05, bevorzugt mindestens 0,1, besonders bevorzugt mindestens 0,15.

Geeignete Monomere i) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Bevorzugt sind saure Gruppen enthaltende Monomere i). Besonders bevorzugte Monomere sind Acrylsäure und Methacrylsäure. Ganz besonders bevorzugt ist Acrylsäure.

Die Monomere i), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden 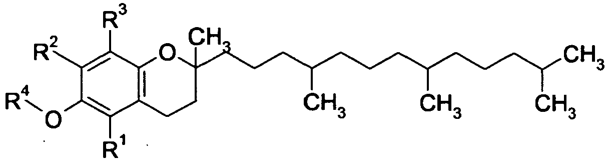 wobei R¹ Wasserstoff oder Methyl, R² Wasserstoff oder Methyl, R³ Wasserstoff oder Methyl und R⁴ Wasserstoff oder ein Säurerest mit 1 bis 20 Kohlenstoffatomen bedeutet.

Bevorzugte Reste für R⁴ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R¹ = R² = R³ = Methyl, insbesondere racemisches alpha-Tocopherol. R⁴ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere bevorzugt um 50 Gew.-ppm, Hydrochinonhalbether, jeweils bezogen auf Acrylsäure, wobei Acrylsäuresalze rechnerisch als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die im erfindungsgemäßen Verfahren einsetzbaren hygroskopischen Substanzen ii) wurden bereits oben als hygroskopische Substanzen b) beschrieben.

Die wasserabsorbierenden Polymere sind vernetzt, d.h. die Polymerisation wird in Gegenwart von Verbindungen mit mindestens zwei polymerisierbaren Gruppen, die in das Polymernetzwerk radikalisch einpolymerisiert werden können ("Vernetzer"), durchgeführt, so dass das hergestellte Polymer einen entsprechenden Anteil des Vernetzers enthält, selbstverständlich in einpolymerisierter Form. Geeignete Vernetzer iii) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 530 438 beschrieben, Di- und Triacrylate, wie in EP-A 547 847, EP-A 559 476, EP-A 632 068, WO 93/21237, WO 03/104299, WO 03/104300, WO 03/104301 und in DE-A 103 31 450 beschrieben, gemischte Acrylate, die neben Acrylatgruppen weitere ethylenisch ungesättigte Gruppen enthalten, wie in DE-A 103 314 56 und der älteren deutschen Anmeldung mit dem Aktenzeichen 103 55 401.7 beschrieben, oder Vernetzermischungen, wie beispielsweise in DE-A 195 43 368, DE-A 196 46 484, WO 90/15830 und WO 02/32962 beschrieben.

Geeignete Vernetzer iii) sind insbesondere N,N'-Methylenbisacrylamid und N,N'Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 343 427 beschrieben sind. Weiterhin geeignete Vernetzer iii) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer iii) sind jedoch Di- und Triacrylate des 3- bis 20-fach ethoxylierten Glyzerins, des 3- bis 20-fach ethoxylierten Trimethylolpropans, des 3- bis 20-fach ethoxylierten Trimethylolethans, inbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des mindestens 40-fach ethoxylierten Glyzerins, Trimethylolethans oder Trimethylolpropans.

Ganz besonders bevorzugte Vernetzer iii) sind die mit Acrylsäure oder Methacrylsäure zu Di- oder Triacrylaten veresterten mehrfach ethoxylierten und/oder propoxylierten Glyzerine wie sie beispielsweise in WO 03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Der Vernetzer wird der Monomerlösung in einer üblichen Menge von beispielsweise 0,5 bis 5,5 Gew.-Teilen bezogen auf 100 Gew.-Teile Monomer i) zugegeben. In einer besonders bevorzugten Ausführungsform des Verfahrens wird er jedoch in einer Menge von im mindestens 6 Gew.-Teile, vorzugsweise mindestens 7 Gew.-Teile, in besonders bevorzugter Weise mindestens 8 Gew.-Teile und in ganz besonders bevorzugter Weise mindestens 9 Gew.-Teile zugegeben, jeweils bezogen auf die 100 Gew.-Teile Monomer i). Die Obergrenze des Gehalts an Vernetzer im Monomer ist weniger kritisch, im Allgemeinen wird ein Gehalt von höchstens 25 Gew.-Teile, vorzugsweise höchstens 20 Gew.-Teile und in besonders bevorzugter Weise von höchstens 18 Gew.-Teile eingestellt. Beispiele geeigneter Vernetzergehalte sind mindestens 10 Gew.-Teile, mindestens 10,5 Gew.-Teile, mindestens 11 Gew.-Teile, mindestens 11,5 Gew.-Teile, mindestens 12 Gew.-Teile, mindestens 12,5 Gew.-Teile, mindestens 13 Gew.-Teile bezogen auf 100 Gew.-Teil Monomer i).

Mit den Monomeren i) copolymerisierbare ethylenisch ungesättigte Monomere iv) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Gegebenenfalls enthält die Monomerlösung ein oder mehrere wasserlösliche Polymere v). Als wasserlösliche Polymere v) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Hygroskopische Polymere, wie lösliche Polyacrylsäuren, können sowohl als hygroskopische Substanz ii) als auch als wasserlösliches Polymer v) eingesetzt werden.

Werden der Monomerlösung übliche Pfropfpolymerisationskatalysatoren, beispielsweise Eisensalze, zugesetzt, so werden die Polymere als Pfropfgrundlage für die Polymerisation dienen und die polymerisierenden Monomere auf die Polymeren aufgepfropft werden. Wird auf den Einsatz von Pfropfpolymerisationskatalysatoren verzichtet, so werden die Polymeren die Polymerisation weitgehend unverändert überstehen und als hygroskopische Substanz wirken.

Die Säuregruppen der bevorzugten Monomere i) sind üblicherweise teilweise neutralisiert, vorzugsweise zu 25 bis 95 mol-%, bevorzugt zu 40 bis 90 mol-%, besonders bevorzugt zu 50 bis 80 mol-%, ganz besonders bevorzugt zu 60 bis 80 mol%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.% als wachsartige Masse mit einem Schmelzpunkt oberhalb 23°C vorliegen. In diesem Fall ist eine Dosierung als Stückgut oder Schmelze bei erhöhter Temperatur möglich.

Die wässrige Monomerlösung wird auf das Trägermaterial aufgebracht, vorzugsweise aufgesprüht. Die geeigneten Trägermaterialien wurden bereits oben als Trägermaterial a) beschrieben.

Anschließend wird die Monomerlösung auf dem Trägermaterial polymerisiert und der Verbundstoff getrocknet. Die Polymerisation wird vorzugsweise durch UV-Strahlung und/oderthermisch induziert. Wie üblich bei derartigen Polymerisationsreaktionen kann zur Beschleunigung oder Kontrolle des Starts der Polymerisationsreaktion ein Initiator verwendet werden, hier ist die Verwendung jedes bekannten Initiators oder Initiatorsystems in üblicher Weise möglich.

Die Polymere c) enthalten mindestens einen Weichmacher, der der Monomerlösung vor der Polymerisation zugesetzt wird und im Polymeren verbleibt. Unter "Weichmacher" wird im Zusammenhang mit dieser Erfindung eine Substanz verstanden, die in der angewendeten Menge die Glasübergangstemperatur des Polymeren c) absenkt. Im Allgemeinen senkt der Weichmacher in der zu verwendenden Menge die Glasübergangstemperatur des Polymeren um mindestens 2°C, vorzugsweise mindestens 4°C, in besonders bevorzugter Weise um mindestens 6°C und in ganz besonders bevorzugter Weise um mindestens 10°C ab. Beispielsweise wird ein Weichmacher in einer Menge zugesetzt, die die Glasübergangstemperatur des Polymeren um mindestens 20°C oder um mindestens 30°C absenkt. Die Glasübergangstemperatur ist eine bekannte Eigenschaft von Polymeren und kann nach der Methode ASTM E1356-03 "Standard Test Method for Assignment of the Glass Transition Temperatures by Differential Scanning Calorimetry" oder der äquivalenten Norm ISO 11357-2 gemessen werden.

Übliche Weichmacher sind bei Raumtemperatur flüssig und sind auch Lösungs- oder Dispersionsmittel für das Polymere. Sofern das Polymere durch UV-Bestrahlung aus dem Monomeren erzeugt wird, ist ein gegen UV-Licht ausreichend stabiler Weichmacher zu wählen, der zudem die UV-induzierte Polymerisation nicht stört. Vorzugsweise ist der Weichmacher hydrophil und mit Wasser unbegrenzt mischbar. Geeignete Weichmacher sind beispielsweise Alkohole, Polyalkohole wie Glycerin und Sorbit, Glykole und Etherglykole wie Mono- oder Diether von Polyalkylenglykolen, Mono- oder Diester von Polyalkylengklykolen, Polyethylenglykole, Polypropylenglykole, gemischte Polyethylen- und Propylenglyklole, Glykolate, Glycerol, Sorbitanester, Zitronen- und Weinsäureester oder vom Imidazolin abgeleitete amphotere Tenside. Bevorzugte Weichmacher sind Polyalkohole wie Glycerin und Sorbit, Polyethylenglykol und ihre Mischungen. Besonders bevorzugt ist Glycerin.

Als Weichmacher auch geeignet sind Kohlenwasserstoffe und Kohlenwasserstoffgemische wie Weißöl, insbesondere medizinisches Weißöl oder Flüssigparaffin.

Der Weichmacher wird üblicherweise in einer Menge zugesetzt, die zum Erreichen der gewünschten Absenkung der Glasübergangstemperatur ausreicht. Typische Gehalte an Weichmacher sind 5 - 50 Gew.-Teile, bevorzugt 8- 40 Gew.-Teile, insbesondere 10 - 30 Gew.-Teile Weichmacher bezogen auf 100 Gew.-Teile Monomer i).

Die erfindungsgemäßen Verbundstoffe eignen sich vorzüglich zur Feuchtigkeitsregulierung, insbesondere in Matratzen und Sitzpolstern, beispielsweise in Autositzen.

Sitzpolster oder Matratzen, enthaltend die erfindungsgemäßen Verbundstoffe, erhöhen den Sitz- bzw. Liegekomfort, indem sie die relative Luftfeuchtigkeit auf ein angenehmes Maß regulieren und übermäßiges Schwitzen verhindern. Gleichzeitig können die erfindungsgemäßen Verbundstoffe die aufgenommene Feuchtigkeit in Phasen der Nichtbenutzung optimal wieder abgeben und sich schnell regenerieren. Aufgrund dieses ausgewogenen Eigenschaftsprofils ermöglichen die erfindungsgemäßen Verbundstoffe einen bisher unerreichten Sitz- bzw. Liegekomfort.

### Methoden:

### Bestimmung der Feuchtigkeitsaufnahme

Die Verbundstoffe werden 60 Minuten bei einer Temperatur von 23 °C und einer relativen Luftfeuchtigkeit von 50% im Klimaschrank zur Gleichgewichtseinstellung gelagert. Anschließend wird die relative Luftfeuchtigkeit auf 90% erhöht und die Verbundstoffe für 90 Minuten bei 30 °C gehalten (Absorptionsphase). Danach wird die relative Luftfeuchtigkeit auf 40 % gesenkt und die Probe für 100 Minuten bei 40 °C gehalten (Desorptionsphase).

Die Gewichtsänderung durch Absorption/Desorption wird kontinuierlich gemessen und als Gewichtszunahme, bezogen auf g aufgebrachter Substanz (wasserabsorbierendes Polymer und/oder Salz). Bezugspunkt für die Gewichtszunahme ist das Gewicht nach Gleichgewichtseinstellung nach 60 Minuten.

### Beispiele

### Beispiel 1

Auf ein Polyethylenterephthalt-Vlies mit einem Flächengewicht von 70 g/m² wurde eine Monomerlösung aufgesprüht und 2 Minuten mittels UV-Strahlung gehärtet. Anschließend wurde für 5 Minuten bei 90°C in einem Gegenstromtrockner getrocknet.

Die Monomerlösung enthielt 19599 g einer 37,5 Gew.-%igen wässrigen Natriumacrylatlösung (entsprechend 24,5 Gew.-% Natriumacrylat in der gesamten Monomerlösung), 435 g Acrylsäure (8,5 Gew.-%), 900 g Polyethylenglykoldiacrylat (Diacrylat eines Polyethylenglykols mit einer mittleren Molmasse von 400) (3 Gew.-%) als Vernetzer, 66 g 2-Hydroxy-1-[4-(hydroxyethoxy)phenyl]- 2-methyl-1-propanon (0,22 Gew.-%) als Initiator, 1500 g Glycerin [5 Gew.-%) und 7500 g einer 25 Gew.-%igen wässrigen Natriumchloridlösung (6,25 Gew.-% NaCl).

Die Menge an Monomerlösung wurde so gewählt, dass die Beladung des Polyethylenterephthalat-Vlieses mit aufpolymerisiertem wasserabsorbierendem Polymer 160 g/m² betrug.

## Patentansprüche

1. Feuchtigkeitsregulierende Verbundstoffe, umfassend
a) mindestens ein flächiges Trägermaterial,
b) mindestens eine wasserlösliche hygroskopische Substanz und
c) mindestens ein in Gegenwart der Substanz b) auf das Trägermaterial a) aufpolymerisiertes wasserabsorbierendes Polymer, wobei das Polymer als Vernetzer Verbindungen mit mindestens zwei polymerisierbaren Gruppen aufweist, die radikalisch einpolymerisiert werden können, und
wobei das Gewichtsverhältnis von hygroskopischer Substanz b) zu Polymer c) im Bereich von 0,01 und 1 liegt, **dadurch gekennzeichnet, dass** das Polymer c) zusätzlich einen Weichmacher enthält.

2. Verbundstoff gemäß Anspruch 1, **dadurch gekennzeichnet, dass** das Trägermaterial a) ein Gewebe und/oder Vlies ist.

3. Verbundstoff gemäß Anspruch 1 oder 2, **dadurch gekennzeichnet, dass** die relative Feuchte über einer übersättigten wässrigen Lösung der hygroskopischen Substanz b) im Gleichgewicht bei 20°C mindestens 40% beträgt.

4. Verbundstoff gemäß einem der Ansprüche 1 bis 3, **dadurch gekennzeichnet, dass** die hygroskopische Substanz b) ein anorganisches Salz ist.

5. Verbundstoff gemäß einem der Ansprüche 1 bis 4, **dadurch gekennzeichnet, dass** das Polymer c) saure Gruppen enthält.

6. Verbundstoff gemäß Anspruch 5, **dadurch gekennzeichnet, dass** die sauren Gruppen zu mindestens 25 mol-% neutralisiert sind.

7. Verfahren zur Herstellung feuchtigkeitsregulierender Verbundstoffe, wobei eine Monomerlösung, enthaltend
i) mindestens ein ethylenisch ungesättigtes Monomer,
ii) mindestens eine wasserlösliche hygroskopische Substanz,
iii) einen Vernetzer, der mindestens zwei polymerisierbare Gruppen aufweist, die radikalisch einpolymerisiert werden können, und
iv) gegebenenfalls ein oder mehrere mit den unter i) genannten Monomeren copolymerisierbare ethylenisch und/oder allylisch ungesättigte Monomere, auf ein flächiges Trägermaterial aufgebracht und polymerisiert wird, wobei das Gewichtsverhältnis von hygroskopischer Substanz ii) zu Monomer i) zwischen 0,01 und 1 beträgt, das **dadurch gekennzeichnet** ist, dass der Monomerlösung zusätzlich ein Weichmacher zugesetzt wird.

8. Verfahren gemäß Anspruch 7, **dadurch gekennzeichnet, dass** das Monomer i) saure Gruppen enthält.

9. Verfahren gemäß Anspruch 8, **dadurch gekennzeichnet, dass** die sauren Gruppen zu mindestens 25 mol-% neutralisiert sind.

10. Verfahren gemäß einem der Ansprüche 7 bis 9, **dadurch gekennzeichnet, dass** die relative Feuchte über einer übersättigten wässrigen Lösung der hygroskopischen Substanz ii) im Gleichgewicht bei 20°C mindestens 40% beträgt.

11. Verfahren gemäß einem der Ansprüche 7 bis 10, **dadurch gekennzeichnet, dass** die hygroskopische Substanz ii) ein anorganisches Salz ist.

12. Verfahren gemäß einem der Ansprüche 7 bis 11, **dadurch gekennzeichnet, dass** das Trägermaterial ein Gewebe und/oder Vlies ist.

13. Verwendung von Verbundstoffen gemäß einem der Ansprüche 1 bis 6 zur Feuchtigkeitsregulierung.

14. Sitzpolster oder Matratzen, enthaltend Verbundstoffe gemäß einem der Ansprüche 1 bis 6.

## Claims

1. A moisture-regulating composite comprising
a) at least one sheetlike substrate material,
b) at least one water-soluble hygroscopic substance, and
c) at least one water-absorbing polymer polymerized onto said substrate material a) in the presence of said substance b), wherein the polymer comprises a crosslinker comprising compounds having at least two polymerizable groups capable of being free-radically interpolymerized, and
the weight ratio of said hygroscopic substance b) to said polymer c) being in the range of 0.01 to 1, wherein said polymer c) additionally comprises a plasticizer.

2. The composite according to claim 1 wherein said substrate material a) is a woven fabric and/or nonwoven.

3. The composite according to claim 1 or 2 wherein the relative humidity above a supersaturated aqueous solution of said hygroscopic substance b) in equilibrium at 20°C is at least 40%.

4. The composite according to any one of claims 1 to 3 wherein said hygroscopic substance b) is an inorganic salt.

5. The composite according to any one of claims 1 to 4 wherein said polymer c) comprises acidic groups.

6. The composite according to claim 5 wherein said acidic groups are at least 25 mol% neutralized.

7. A process for producing a moisture-regulating composite, which comprises a monomer solution comprising
i) at least one ethylenically unsaturated monomer,
ii) at least one water-soluble hygroscopic substance,
iii)a crosslinker having at least two polymerizable groups capable of being free-radically interpolymerized, and,
iv) optionally one or more ethylenically and/or allylically unsaturated monomers copolymerizable with the monomers mentioned under i),
being applied to a sheetlike substrate material and polymerized, the weight ratio of said hygroscopic substance ii) to said monomer i) being between 0.01 and 1, said process further comprising a plasticizer being additionally included in said monomer solution.

8. The process according to claim 7 wherein said monomer i) comprises acidic groups.

9. The process according to claim 8 wherein said acidic groups are at least 25 mol% neutralized.

10. The process according to any one of claims 7 to 9 wherein the relative humidity above a supersaturated aqueous solution of said hygroscopic substance ii) in equilibrium at 20°C is at least 40%.

11. The process according to any one of claims 7 to 10 wherein said hygroscopic substance ii) is an inorganic salt.

12. The process according to any one of claims 7 to 11 wherein said substrate material is a woven fabric and/or nonwoven.

13. Use of a composite according to any one of claims 1 to 6 for moisture regulation.

14. Seat padding or a mattress comprising a composite according to any one of claims 1 to 6.

## Revendications

1. Composites régulant l'humidité, comprenant .
a) au moins un matériau support plat,
b) au moins une substance hygroscopique soluble dans l'eau et
c) au moins un polymère absorbant l'eau polymérisé sur le matériau support a) en présence de la substance b), le polymère comprenant en tant qu'agent de réticulation des composés contenant au moins deux groupes polymérisables qui peuvent être polymérisés par voie radicalaire, et
le rapport en poids entre la substance hygroscopique b) et le polymère c) se situant dans la plage allant de 0,01 à 1, **caractérisés en ce que** le polymère c) contient en outre un plastifiant.

2. Composite selon la revendication 1, **caractérisé en ce que** le matériau support a) est un tissu et/ou un non-tissé.

3. Composite selon la revendication 1 ou 2, **caractérisé en ce que** l'humidité relative par le biais d'une solution aqueuse sursaturée de la substance hygroscopique b) à l'équilibre à 20 °C est d'au moins 40 %.

4. Composite selon l'une quelconque des revendications 1 à 3, **caractérisé en ce que** la substance hygroscopique b) est un sel inorganique.

5. Composite selon l'une quelconque des revendications 1 à 4, **caractérisé en ce que** le polymère c) contient des groupes acides.

6. Composite selon la revendication 5, **caractérisé en ce que** les groupes acides sont neutralisés à au moins 25 % en moles.

7. Procédé de fabrication de composites régulant l'humidité, selon lequel une solution de monomères contenant :
i) au moins un monomère éthyléniquement insaturé,
ii) au moins une substance hygroscopique soluble dans l'eau,
iii) un agent de réticulation contenant au moins deux groupes polymérisables qui peuvent être polymérisés par voie radicalaire, et
iv) éventuellement un ou plusieurs monomères éthyléniquement et/ou allyliquement insaturés copolymérisables avec les monomères cités en i),
est appliquée et polymérisée sur un matériau support plat, le rapport en poids entre la substance hygroscopique ii) et le monomère i) étant compris entre 0,01 et 1, **caractérisé en ce qu'**un plastifiant est en outre ajouté à la solution de monomères.

8. Procédé selon la revendication 7, **caractérisé en ce que** le monomère i) contient des groupes acides.

9. Procédé selon la revendication 8, **caractérisé en ce que** les groupes acides sont neutralisés à au moins 25 % en moles.

10. Procédé selon l'une quelconque des revendications 7 à 9, **caractérisé en ce que** l'humidité relative par le biais d'une solution aqueuse sursaturée de la substance hygroscopique ii) à l'équilibre à 20 °C est d'au moins 40 %.

11. Procédé selon l'une quelconque des revendications 7 à 10, **caractérisé en ce que** la substance hygroscopique ii) est un sel inorganique.

12. Procédé selon l'une quelconque des revendications 7 à 11, **caractérisé en ce que** le matériau support est un tissu et/ou un non-tissé.

13. Utilisation de composites selon l'une quelconque des revendications 1 à 6 pour la régulation de l'humidité.

14. Garnitures de sièges ou matelas, contenant des composites selon l'une quelconque des revendications 1 à 6.
